(19)

Europäisches
Patentamt
European
Patent Office
Office européen
des brevets

(11) **EP 1 343 872 B1**

(12) **EUROPEAN PATENT SPECIFICATION**

(45) Date of publication and mention
of the grant of the patent:
**14.08.2013  Bulletin 2013/33**

(21) Application number: **01272072.8**

(22) Date of filing: **19.12.2001**

(51) Int Cl.:
***C12N 5/22*** *(2006.01)*

(86) International application number:
**PCT/GB2001/005606**

(87) International publication number:
**WO 2002/051985 (04.07.2002 Gazette 2002/27)**

(54) **CULTURING TISSUE USING MAGNETICALLY GENERATED MECHANICAL STRESSES**

GEWEBEKULTUR UNTER MAGNETISCH AUSGELÖSTEM MECHANISCHEM STRESS

CULTURE DE TISSUS AU MOYEN DE CONTRAINTES MECANIQUES GENEREES DE MANIERE MAGNETIQUE

(84) Designated Contracting States:
**AT BE CH CY DE DK ES FI FR GB GR IE IT LI LU MC NL PT SE TR**

(30) Priority: **22.12.2000  GB 0031462**
**23.12.2000  GB 0031651**
**07.02.2001  US 267032 P**

(43) Date of publication of application:
**17.09.2003  Bulletin 2003/38**

(73) Proprietor: **MICA BioSystems Limited**
**Solihull**
**West Midlands B91 1NJ (GB)**

(72) Inventors:
• **EL-HAJ, Alicia, Jennifer, Hafeeza**
**New Mills,**
**High Peak SK22 4EQ (GB)**
• **DOBSON, Jon, Paul**
**Endon,Stoke-on-Trent ST9 9EZ (GB)**

(74) Representative: **Clegg, Richard Ian**
**Mewburn Ellis LLP**
**33 Gutter Lane**
**London**
**EC2V 8AS (GB)**

(56) References cited:
**US-A- 5 486 457**

• **YUGE LOUIS ET AL: "Differentiation of myoblasts is accelerated in culture in a magnetic field." IN VITRO CELLULAR & DEVELOPMENTAL BIOLOGY ANIMAL, vol. 36, no. 6, June 2000 (2000-06), pages 383-386, XP001061490 ISSN: 1071-2690**
• **LIU MINGYAO ET AL: "Bio-stretch, a computerized cell strain apparatus for three-dimensional organotypic cultures." IN VITRO CELLULAR & DEVELOPMENTAL BIOLOGY ANIMAL, vol. 35, no. 2, February 1999 (1999-02), pages 87-93, XP001080069 ISSN: 1071-2690**
• **KASPAR D ET AL: "Dynamic cell stretching increases human osteoblast proliferation and CICP synthesis but decreases osteocalcin synthesis and alkaline phosphatase activity." JOURNAL OF BIOMECHANICS, vol. 33, no. 1, January 2000 (2000-01), pages 45-51, XP002203098 ISSN: 0021-9290 -& BOTTLANG M ET AL: "A cell strain system for small homogeneous strain applications." BIOMEDIZINISCHE TECHNIK, vol. 42, no. 11, November 1997 (1997-11), pages 305-309, XP001080015 ISSN: 0013-5585**

**(Cont. next page)**

- GLOGAUER MICHAEL ET AL: "A new method for application of force to cells via ferric oxide beads." PFLUEGERS ARCHIV EUROPEAN JOURNAL OF PHYSIOLOGY, vol. 435, no. 2, January 1998 (1998-01), pages 320-327, XP002203099 ISSN: 0031-6768 -& DATABASE BIOSIS [Online] BIOSCIENCES INFORMATION SERVICE, PHILADELPHIA, PA, US; 1995 GLOGAUER,M. ET AL.: "Magnetic fields applied to collagen-coated ferric oxide beads induce stretch-activated Ca-2+ flux in fibroblasts." Database accession no. PREV199698605117 XP002203103 & AMERICAN JOURNAL OF PHYSIOLOGY, vol. 269, no. 5 PART 1, 1995, pages C1093-C1104, ISSN: 0002-9513

- SMALT R ET AL: "Induction of NO and prostaglandin E2 in osteoblasts by wall-shear stress but not mechanical strain." AMERICAN JOURNAL OF PHYSIOLOGY, vol. 273, no. 4 PART 1, October 1997 (1997-10), pages E751-E758, XP002203100 ISSN: 0002-9513
- WANG NING ET AL: "Contribution of intermediate filaments to cell stiffness, stiffening, and growth." AMERICAN JOURNAL OF PHYSIOLOGY, vol. 279, no. 1 Part 1, July 2000 (2000-07), pages C188-C194, XP002203101 ISSN: 0002-9513
- WANG NING: "Mechanical interactions among cytoskeletal filaments." HYPERTENSION (DALLAS), vol. 32, no. 1, July 1998 (1998-07), pages 162-165, XP002203102 ISSN: 0194-911X

## Description

The present invention relates to' a method of culturing cells and relates more particularly (but not exclusively) to the culturing of cells to form replacement human or animal tissue. The invention relates even more particularly, but again not exclusively, to the culturing of mechano-responsive tissue.

The *in vitro* cultivation of replacement tissue for humans and animals is an important development allowing the tissue to be grown from cells taken from the patient so that the replacement tissue does not cause rejection problems. Examples of replacement tissues that may be produced for such replacement therapies include connective tissue, bone, cartilage, tendon and pancreas.

The replacement tissue must not only be comprised of the same type of cells as the tissue it is intended to replace but must also have the required, possibly complex, 3-dimensional shape. As such, the replacement tissue is generally grown on or within a suitably shaped scaffold immersed in a culture medium in a bioreactor. The scaffold is a cell growth substrate which is shaped to provide growth of the tissue' into the required 3-dimensional form. Within the bioreactor there is a (generally constant) flow of culture medium ensuring that the tissue-forming cells on or within the scaffold continuously receive a supply of nutrients and that the metabolic waste products of the cells are removed. The typically increased volumes of culture medium that can be used in bioreactors, compared to static culture flasks, allow immersion of scaffolds of a range of sizes suitable for production of a number of different tissue types. The perfusion of culture medium throughout the scaffold allows all cells to benefit from viable conditions for growth throughout the structure [1].

In addition, and particularly in the case of mechano-responsive tissue, it may be necessary to subject the tissue forming cells to mechanical stresses during their culture to produce fully functional tissue. Thus, for example, some types of connective tissue such as bone, cartilage, ligament and tendons need to be subjected to mechanical stress during culturing thereof to give the required mechanical properties [2].

The degree of stress needed varies according to the cell types used and tissue types required. A number of ways of producing such stresses are known in the art, including direct mechanical stimulation of the cells, and hydrodynamic compression systems. The former method uses rollers or the like to compress the cells, whereas the latter utilises pulses of increased pressure in the culture medium supplying the bioreactor to mechanically stimulate the cells. None of the known methods of mechanically stimulating cells in order to produce functional tissue are, however, entirely satisfactory for many types of tissue such as bone, tendon and ligaments. Direct mechanical methods are cumbersome, and produce difficulties in maintaining the aseptic conditions required for culture. Hydrodynamic compression methods are generally ineffective. Moreover, all previously known methods suffer from the disadvantages that only one magnitude of stress can be applied across the range of cultured cells at any one time (generally a much higher stress than is required at a cellular level), and that the scaffold upon which the cells are grown must itself have considerable mechanical resilience to withstand the stresses applied to it.

Yuge Louis et al. (In Vitro Cell Dev. Biol. Animal vol. 36, no.6 June 200 pages 383-386) discloses a cell stimulation method in which magnetic microparticles (MPs) were introduced into the cytoplasm of cultured myoblasts and the cells were cultured in a magnetic field, which provided a precisely quantitative and stable stimulus.

Liu et al. (In Vitro Cell. Dev. Biol. - Anima 33: 87-93, February 1999) discloses a computerized Bio-Stretch system which can apply uniaxial stretch to cells cultured in a three-dimensional environment. In Kaspar et al. (Journal of Biomechanics 33(2000), 45-51), monolayers of subconfluently grown human-bone-derived cells were stretched in rectangular silicon dishes with cyclic predominantly uniaxial movement along their longitudinal axis. The cell substrate consists of rectangular, optically clear, elastic culture dishes, which were subjected to cyclic, homogenous stretching.

In a method disclosed in Glogauer et al. (Pflügers Arch - Eur J Physiol (1998) 435:320-327), collagen-coated ferric oxide beads attach to the surface of cells via integrin receptors, and a constant (i.e., not time varying) magnetic field gradient that is uniform over a relatively large area is applied to produce a constant force.

Smalt et al. (Am. J. Physiol. Endocrinol. Metab. 273: 751-758(1997)) disclose a method in which cells were incubated on polystyrene film and subjected to unidirectional linear strains. The film was attached to two metal bars, which could be moved relative to each other to induce strain in the film. US 5,486,457 discloses a method in which mechanical stresses and deformations are applied directly to cell surface receptors or molecules and measured using a system including a magnetic twisting device in combination with ferromagnetic microbeads coated with ligands for integrins or any other surface receptors.

Wang et al. (Am. J. Physiol. Cell Physiol. 279; p.188-194 (2000)) discloses the use of magnetic cell twisting to apply varying mechanical stress through integrin receptors to cells.

Wang (Hypertension (1998) 32; p.162-165) disclosed the method of magnetic twisting cytometry, by which rotational stress was applied directly to integrin receptors with ferromagnetic beads coated with RGD containing peptide.

Glogauer et al. (Am. J. Physiol. 269; C1093-C1104 (1995)) discloses a magnetic particle-electromagnet model that allows the application of controlled forces to the plasma membrane of substrate-attached fibroblasts. Ferric oxide microparticles were coated with collagen and individual beads were bound to the cells by collagen receptors. For experiments, cells were placed 1 mm from pole of electromagnet. The magnetic flux density and its gradient were relatively uniform at that distance from the pole piece.

It is therefore an object of the present invention to obviate or mitigate the above mentioned disadvantages.

According to a first aspect of the present invention there is provided a method according to claim 1.

Thus in accordance with the invention, magnetically generated stresses are applied to the tissue forming cells so as to ensure that fully functional tissue is produced.

The method of the invention may be applied *in vitro* for the growth of tissue to be implanted in a patient. If effected *in vitro* it is preferred that the tissue forming cells are cultured on or in a 3-dimensional scaffold and preferably also in a bioreactor through which flows a tissue culture medium. Other types of tissue culture vessels may however be used.

The stresses may be generated by a magnetic material capable of generating' a force in response to a magnetic field applied within the bioreactor and transmitting that force to the tissue forming cells being cultured so as to apply the required stresses thereto. In preferred embodiments of the invention, the magnetic material is attached to the tissue forming cells and, for preference, takes the form of micro- or nano-particles, preferably coated magnetic micro- or nano-particles. Alternatively the magnetic material may be a ferrofluid which is inserted into the culture medium. A further possibility is the combined use of magnetic material attached to the cells and a ferrofluid.

Irrespective of the particular magnetic material used, the use of a time-varying magnetic gradient or homogeneous field modulates the movement of the magnetic material and consequently allows stresses to be repeatedly applied to the tissue forming cells. Such stresses may be accurately varied both in their magnitude and direction of application so that the tissue forming cells may be subjected to the required stress forming regime to ensure that fully functional tissue is produced. This can be accomplished by varying the magnetic properties of particles attached to different cells in different regions of the same scaffold (or different scaffolds) or through the use of the spatial variation of field strength in the gradient field.

The magnetic field may be varied at a frequency of, for example, 0.1 to 10 Hz. But, frequencies outside this range can also be used. The magnetic field will typically have a flux density on the order of (but not limited to) 10 mT to 1400 mT.

The magnitude of the stresses applied to the cells will be generally on the order of (but not limited to) 0.1 to 100 piconewtons (pN) and the direction in which the stress is applied may result from a linear, translational motion (due to the gradient, particle does not need to be magnetically blocked) of the magnetic material in the applied magnetic field.

Significant advantages of the present invention are that (as indicated) it is easy to control the direction and magnitude of the applied stresses whilst maintaining aseptic conditions *in vitro* e.g. in a bioreactor Moreover the stresses that are generated at the cellular level are generally small (e.g. a few piconewtons) [3] and as such any scaffolds (on or in which the tissue forming cells are grown) do not need enhanced mechanical properties.

The method of the invention can be used to produce a variety of tissue types in bioreactor which require mechanical loading or activation of mechanosensitive ion channels. These include (but are not limited to) connective tissue such as bone, cartilage, ligament and tendons. Biopsies of the cells to be cultured may be obtained by standardized procedure [4].

It is also possible for the method of the invention to be applied to tissue constructs comprised of tissues of at least two different types, e.g. bone and cartilage. It is also possible to use human mesenchymal stem cells as a source which are differentiated into chondrocytes or bone cells on or within scaffolds.

As mentioned above, a preferred embodiment of the invention involves attachment of magnetic micro- or nano- particles to the tissue forming cells for the purposes of applying the required stresses thereto. The magnetic micro- and nano-particles may be functionalised and attached to the tissue forming cells prior to seeding of the latter onto a scaffold on or in which the tissue is to be grown. Thus, for example, the micro- and nano-particles may be coated with adhesion molecules, e.g. fibronectin and RGD molecules for attachment to the cells.

The micro- and nano- particles (intended to be attached to the cells) will generally be spherical or elliptical and have a diameter in the range 10nm to 10$\mu$m.

The particles for attachment to the cells may be coated or uncoated and single or multi-domain. Examples of suitable particles include, but are not limited to:

(i) Coated magnetic microspheres (d = 4 $\mu$m) available from Spherotech, Inc. These microspheres consist of a magnetically blocked core -coated by a polymer.

(ii) Single-domain, ferrite-doped silica nanoparticles with tunable size (d = 50-300 nm) and narrow size distribution [5].

It is not however essential for the magnetic material to be particulate nor that it be attached to the cells. It is possible, for example, for the profusion medium in a bioreactor or *in vivo* to contain a ferrofluid which is used to generate forces, from an applied magnetic gradient field, to the cells being cultured. The ferrofluid may for example be a PVA/magnetite nanoparticle-based ferrofluid (d = 4-10nm) [6]. It is possible also to use particles attached to the cells in combination with a ferrofluids.

The bioreactor may for example be a modification of an existing bioreactor such as profusion, spinner flask, hydrodynamic compression and rotating vessel systems.

Conveniently the magnetic field is generated outside the tissue culture vessel, and may be provided by a permanent magnet or an electromagnet. In order to generate variable fields, a permanent magnet may be moved relative to the cells being cultured. Thus, in the case of a bioreactor, such movement may for example be longitudinally along the reactor, towards-and-away from the reactor or around the reactor. Any combination of these movements may also be used. In the case of an electromagnet, varying magnetic fields may be generated by provision of appropriate electric current levels to the electromagnet optionally in combination with movement of the electromagnet in the same manner as described for the permanent magnet.

Examples of commercially available magnets that may be used include Neodymium-Iron-Boron and Samarium-Cobalt permanent magnets that are capable of generating the required field gradients and flux densities. They can be geometrically tailored and magnetized to a variety of required specifications and produce flux densities at the surface in excess of 1 T (10,000 Gauss). Examples of electromagnets that may be used include cryo-cooled, superconducting magnetic coils capable of producing fields of several tesla.

The forces applied to the cells will generally be from 0.1 to lOpN (as previously indicated), such forces being capable of opening transmembrane ion channels. The magnetic fields and field gradients required to generate these forces vary depending on the magnetic, volumetric and shape properties of the particles and the distance between the tissue construct and the magnet. These parameters are governed by the equation:

$$F_{mag} = (\chi_2 - \chi_1)V\frac{1}{\mu_o}B(\nabla B)$$

where $\chi_2$ is the volume magnetic susceptibility of the magnetic particle, $\chi_1$ is the volume magnetic susceptibility of the surrounding medium (i.e. tissue/bone), $\mu_0$ is the magnetic permeability of free space, **B** is the magnetic flux density in Tesla (T). Though this assumes spherical particles and no magnetic dipole interactions, it should give a good approximation of the field and gradient required for the system.

As the value of $\chi_1$ for human tissue is very small and negative in comparison with the magnetic susceptibility of magnetite (or other magnetic material which will be used in the ferrofluids, and nanoparticles), $\chi_1$ is negligible for this calculation and the expression ($\chi_2$ - $\chi_1$) can be reduced to $\chi_2$. Also, as we are interested in the translational motion of the magnetite particle/fluid/material in an applied field along the z-axis (vertical) and, assuming a relative permeability of 1, the force expression can be reduced to:

$$F_{mag} = (\chi_2)VB\frac{dB}{dz}$$

for particles close to the magnetic field source.

It can be seen from these equations that the compressional (translational) force experienced by the tissue constructs in the presence of ferrofluids and magnetic particles is dependent on the strength of the field, the field gradient and the volumetric and magnetic properties of the particles. One of these parameters will have a strong spatial variation - the field strength/gradient product. This will enable the application of differential forces in three dimensions. In addition, by seeding different regions of the scaffold with particles, ferrofluids and magnetic materials of differing magnetic and volumetric properties, the three-dimensional variation in applied force can be enhanced. This facilitates the growth of complex tissue structures via the spatial variation of applied forces inside the bioreactor.

A number of different scaffold types (essentially 3-dimensional porous blocks which can be varied in dimension) can be used. One of the advantages of the present invention is that the scaffolds do not need enhanced mechanical properties as the stresses involved are generally small. It is possible, for example, to use a biodegradable, porous polylactic acid (PLA) based scaffold. Alternative scaffolds include PGA (poly glycolic acid) materials which are rapidly degrading and are less mechanically strong and collagen scaffolds which are natural materials [7]. The scaffold may be coated with collagen type 1 or other adhesion molecules (such as RGD or non-RGD based molecules) to improve cell adhesion.

The invention will be illustrated, by way of example only, with reference to the accompanying drawings, in which:

Fig 1 illustrates a first embodiment of the invention;

Fig 2 illustrates a second embodiment of the invention; and

Fig 3 illustrates activation of a mechano-sensitive transmembrane ion channel by the use of a magnetic field.

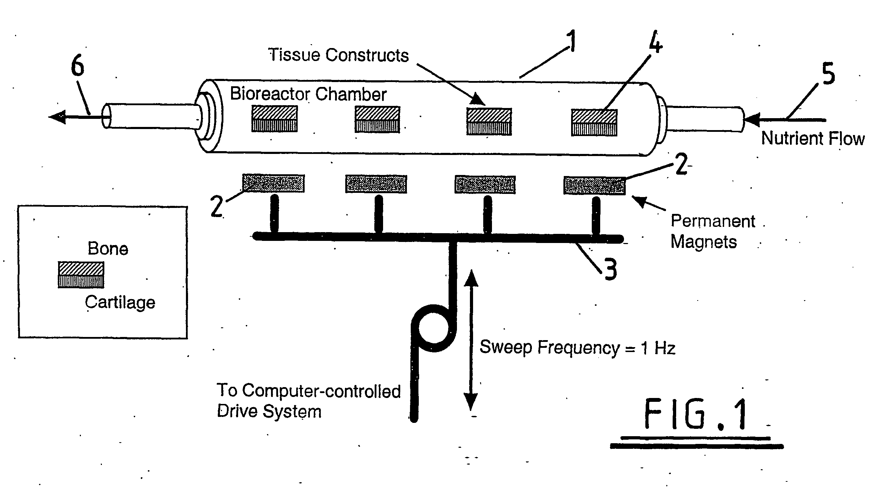

Referring to Fig 1, there is illustrated a tubular bioreactor 1 associated with permanent magnets 2 which are positioned externally of the reactor 1 and which are mounted on a carrier arrangement 3 connected to a computer-controlled (or other time-varying) drive system not illustrated in detail in the drawings.

Within the bioreactor 1 are a number of longitudinally spaced tissue constructs 4 each depicted as being comprised on bone tissue on one side (the side remote from the magnets 3) and cartilage tissue on the other side (other tissue types may also be used). The tissue cells have magnetic beads (not shown) attached thereto and are seeded on 3-D scaffolds (again not shown). Nutrients are supplied to the bioreactor as depicted by arrow 5 and exit therefrom as depicted by arrow 6.

There are a total of four magnets 2 (though this number can vary to match the number of tissue constructs) which are external of the bioreactor and longitudinally spaced therealong. The positioning of the magnets is such that there is one magnet associated with each of the tissue constructs, the magnets being provided on the cartilage sides thereof.

In use of the apparatus, the carrier is driven so as to oscillate the magnets transversely towards and away from the bioreactor. The oscillation frequency at which the magnets are driven will usually be varied and generally be in the range of 0.1 to 10 Hz although values outside this range may be used.

The oscillation of the magnets stimulates a compression/relaxation cycle which is applied to the tissue constructs, the frequency of which can also be varied by mechanical drivers (not shown) attached to the magnets. The magnet field gradient (spatially varying magnetic field strength) ensures that the cartilage experiences slightly higher flux densities than the bone cells.

Strong magnetic field gradients will produce a translational motion on the nanoparticles directed towards the magnets, compressing the cells and scaffold inside the bioreactor. This compression will simulate mechanical loading without requiring direct access to the cells inside the bioreactor. Loads can be easily varied by changing the magnetic field strength and gradient, magnet position and/or the physical properties of the nanoparticles compressing the tissue constructs.

If desired, the magnetic particles associated with the bone cells may have different magnetic properties from those associated with the cartilage so that different mechanically stresses are applied to the two different types of cells.

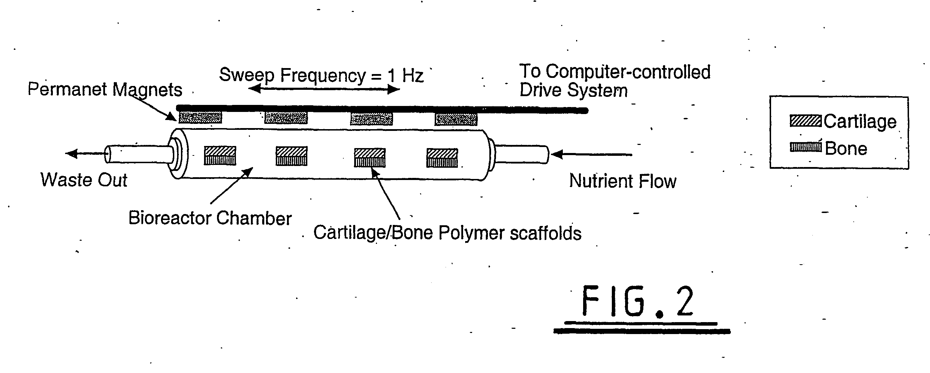

Fig 2 illustrates a modification of the apparatus shown in Fig 1. In the modification of Fig 2, the (permanent) magnets are oscillated parallel to the longitudinal axis of the bioreactor (rather then transversely to the axis in the case of Fig 1).

A number of modifications may be made to the illustrated embodiments.

Thus, for example, the magnets may be swept relatively around the bioreactor. This may most conveniently, but not necessarily, be achieved by keeping the magnets fixed and rotating the bioreactor around its longitudinal axis.

Alternatively or additionally the permanent magnets illustrated in Figs 1 and 2 may be replaced by electromagnets. A further possibility is for the nanoparticles attached to the cells to be replaced by ferrofluids. If desired, a combination of attached nanoparticles and ferrofluids may also be used.

A further possibility is to use magnetic/metal plates or other structures which could, be attracted to the magnets in order to deform the entire scaffold.

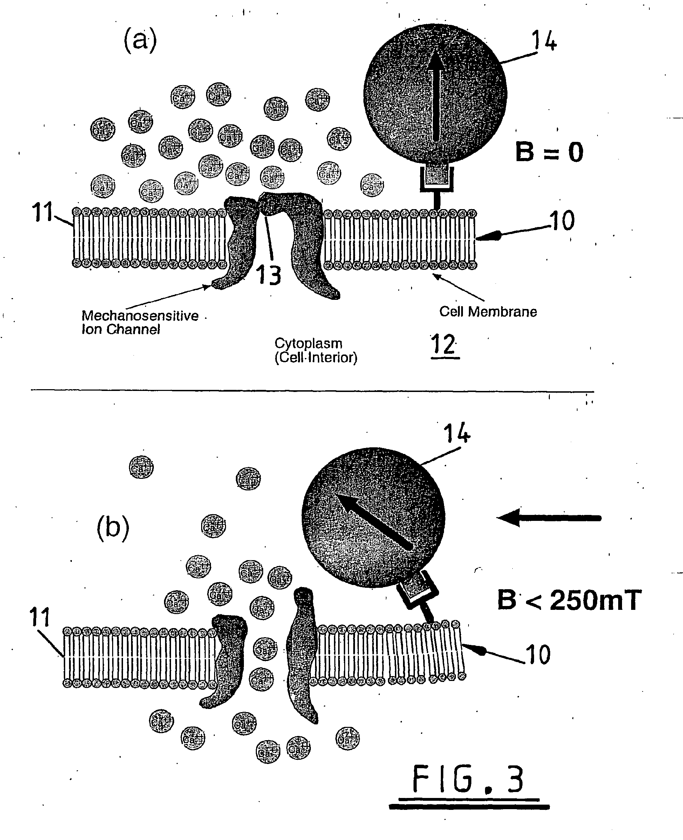

Reference is now made to Fig 3 which illustrates an alternative methods of activation of mechano-sensitive transmembrane ion channels by the use of a magnetic field so as to simulate period mechanical loading of a tissue construct.

More particularly, Fig 3 illustrates a cell 10 having a membrane 11 enclosing the cell's cytoplasm 12. Within membrane 11 is a mechanosensitive ion channel 13. A functionalized magnetically blocked particle 14 (such as Sphereotech's coated ferromagnetic particles, $d=4.5\mu m$) is rigidly attached to the cell membrane 11 either directly or indirectly via cytoskeletal coupling [8, 9].

In the condition shown in Fig 3(a), no magnetic field is applied to the cell 10 and the ion channel 13 is closed.

By oscillating a magnetic field source (not shown) the magnetic particle 14 attached to the cell 10 can be twisted, exerting a mechanical stress on the cell membrane 11 and activating the mechano-sensitive in channel 13 (Figure 3b). This ion channel activation initiates biochemical reaction pathways in the cells being cultured and simulates periodic mechanical loading of tissue constructs inside the bioreactor.

**REFERENCES:**

1. Ying, Y., Peak, M., Magnay, J., and El Haj, A.J. (2000) Dynamic cell scaffold interactions: implications for tissue engineering. Proceedings of the second Smith and Nephew international symposium on tissue engineering, York, UK.
2. El Haj, AJ, LM Walker, MR Preston, SJ Publicover (1999) Mechanotransduction pathways in bone: calcium fluxes and the role of voltage-operated calcium channels. Med. Biol. Eng. Comp., 37: 403-409.
3. Howard J and AJ Hudspeth (1989) Compliance of the hair bundle associated with the gating of mechanoelectrical transduction channels in the bullfrog's saccular hair cell. Neuron 1: 189-199.
4. Walker et al J Cell Biochem 2000.
5. Pardoe, H, W Chua-anusorn, TG St. Pierre, J Dobson (2000) Structural and magnetic properties of nanoscale magnetic particles synthesised by coprecipitation of iron oxide in the presence of dextran or polyvinyl alcohol. J. Magn. Mag. Materials. In Press.
6. Tan, W, S Santra, Z Peng, R Tapec and J Dobson (2000) Coated nanoparticles. *US Patent Pending* (Filed May 17, 2000).

7. Sittinger et al 1996.

8. Kirschvink, JL (1992) Comments on "Constraints on biological effects of weak extremely-low-frequency electromagnetic fields". Phys. Rev. A. 46: 2178-2184.

9. Dobson, J and TG St. Pierre (1996) Application of the Ferromagnetic Transduction Model to D.C. and Pulsed Magnetic Fields: Effects on Epileptogenic Tissue and Implications for Cellular Phone Safety. Biochem. Biophys. Res. Commun., 227:718-723.

**Claims**

1. An in vitro method of culturing tissue, the method comprising the steps of:

    i) applying a magnetic material, capable of generating a force in response to an applied magnetic field, to tissue-forming cells;
    ii) subjecting the magnetic material to a magnetic field so that the force generated by the magnetic material is transmitted to the tissue-forming cells, thereby subjecting the tissue-forming cells to mechanical stresses;

    **characterised in that** the applied magnetic field is varied in a linear translational direction such that a linear translational force is applied relative to the tissue-forming cells, and **in that** the magnetic material comprises micro- or nano-magnetic particles or a ferrofluid.

2. A method as claimed in claim 1 wherein the magnetic material is attached to the tissue forming cells.

3. A method as claimed in claim 1 or claim 2, wherein the magnetic field is varied sinusoidally.

4. A method as claimed in claims 1 to 3, wherein the magnetic field is varied at a frequency of 0.1 to 10Hz.

5. A method as claimed in claims 1 to 4 wherein the tissue forming cells are grown on or in a 3-dimensional scaffold.

6. A method as claimed in claims 1 to 5 wherein the tissue forming cells are cultured in a bioreactor (1) through which flows a culture medium.

7. A method as claimed in claim 6 wherein the magnetic field is applied from externally of the bioreactor (1).

8. A method as claimed in any one of claims 1 to 7, wherein the cells being cultured are for forming connective tissue.

9. A method as claimed in claim 8, wherein the cells being cultured are for forming bone, cartilage, ligament or tendons.

10. A method as claimed in any one of claims 1 to 9, wherein two or more different cell types are cultured.

11. A method as claimed in claim 10, wherein the cells being cultured comprise bone-forming cells and cartilage-forming cells.

12. A method as claimed in any one of claims 1 to 11, wherein the forces applied to the cells or scaffold are in the range 0.1 to 100 pN.

13. A method as claimed in claim 6 wherein the magnetic field is generated outside the tissue culture vessel, wherein the magnetic field is provided by a permanent magnet (2) or an electromagnet, wherein the permanent magnet is moved relative to the cells being cultured, such movement being longitudinally along the bioreactor (1) or towards and away from the bioreactor (1).

14. A method as claimed in claim 6 wherein permanent magnets or electromagnets (2) are positioned externally of the bioreactor (1) and are longitudinally spaced therealong, the magnets (2) being mounted on a carrier arrangement driven to oscillate the magnets (2) transversely towards and away from the bioreactor (1).

15. A method as claimed in claim 6 wherein permanent magnets or electromagnets (2) are positioned externally of the bioreactor (1) and are longitudinally spaced therealong, the magnets (2) being mounted on a carrier arrangement driven so as to oscillate the magnets (2) parallel to the longitudinal axis of the bioreactor (1).

**Patentansprüche**

1. In-vitro-Verfahren zur Kultivierung von Gewebe, wobei das Verfahren die folgenden Schritte umfasst:

    i) Anlegen eines magnetischen Materials, das in der Lage ist, als Reaktion auf ein angelegtes Magnetfeld eine Kraft zu erzeugen, an gewebsbildende Zellen;
    ii) Aussetzen des magnetischen Materials gegenüber einem Magnetfeld, sodass die durch das magnetische Material erzeugte Kraft an die gewebsbildenden Zellen übertragen wird, wodurch die gewebsbildenden Zellen mechanischer Beanspruchung ausgesetzt werden;

    **dadurch gekennzeichnet, dass** das angelegte Magnetfeld in lineare Translationsrichtung variiert, sodass eine lineare Translationskraft in Bezug auf die gewebsbildenden Zellen angelegt wird, und dass das magnetische Material magnetische Mikro- oder Nanopartikel oder ein Ferrofluid ist.

2. Verfahren nach Anspruch 1, wobei das magnetische Material an die gewebsbildenden Zellen gebunden ist.

3. Verfahren nach Anspruch 1 oder Anspruch 2, wobei das magnetische Feld sinusförmig variiert.

4. Verfahren nach einem der Ansprüche 1 bis 3, wobei das Magnetfeld mit einer Frequenz von 0,1 bis 10 Hz variiert.

5. Verfahren nach einem der Ansprüche 1 bis 4, wobei die gewebsbildenden Zellen auf oder in einem 3-dimensionalen Gerüst gezüchtet werden.

6. Verfahren nach einem der Ansprüche 1 bis 5, wobei die gewebsbildenden Zellen in einem Bioreaktor (1) kultiviert werden, durch den ein Kulturmedium fließt.

7. Verfahren nach Anspruch 6, wobei das Magnetfeld von außerhalb des Bioreaktors (1) angelegt wird.

8. Verfahren nach einem der Ansprüche 1 bis 7, wobei die kultivierten Zellen zur Bildung von Bindegeweben dienen.

9. Verfahren nach Anspruch 8, wobei die Zellen zur Bildung von Knochen, Knorpel, Bändern oder Sehnen dienen.

10. Verfahren nach einem der Ansprüche 1 bis 9, wobei zwei oder mehr unterschiedliche Zelltypen kultiviert werden.

11. Verfahren nach Anspruch 10, wobei die kultivierten Zellen knochenbildende Zellen und knorpelbildende Zellen umfassen.

12. Verfahren nach einem der Ansprüche 1 bis 11, wobei die an die Zellen oder das Gerüst angelegten Kräfte im Bereich von 0,1 bis 100 pN liegen.

13. Verfahren nach Anspruch 6, wobei das Magnetfeld außerhalb des Gewebekulturbehälters erzeugt wird, wobei das Magnetfeld durch einen Permanentmagnet (2) oder einen Elektromagnet bereitgestellt wird, wobei der Permanentmagnet in Bezug auf die kultivierten Zellen bewegt wird, wobei die Bewegung in Längsrichtung des Bioreaktors (1) oder zum Bioreaktor (1) hin und von diesem Weg erfolgt.

14. Verfahren nach Anspruch 6, wobei Permanentmagneten oder Elektromagneten (2) außerhalb des Bioreaktors (1) positioniert sind und dessen Länge nach voneinander beabstandet sind, wobei die Magneten (2) auf einer Trägeranordnung angeordnet sind, die zum Schwingen der Magneten (2) transversal zum Bioreaktor (1) hin und von diesem Weg betrieben wird.

15. Verfahren nach Anspruch 6, wobei die Permanentmagneten oder Elektromagneten (2) außerhalb des Bioreaktors (1) angeordnet sind und dessen Länge nach voneinander beabstandet sind, wobei die Magneten (2) auf einer Trägeranordnung angeordnet sind, die zum Schwingen der Magneten (2) parallel zur Längsachse des Bioreaktors (1) betrieben wird.

**Revendications**

1. Procédé *in vitro* de culture d'un tissu, le procédé comprenant les étapes qui consistent à :

    i) appliquer un matériau magnétique, capable de générer une force en réponse à un champ magnétique appliqué, à des cellules formant un tissu ;
    ii) soumettre le matériau magnétique à un champ magnétique de sorte que la force générée par le matériau magnétique soit transmise aux cellules formant un tissu, ce qui soumet ainsi les cellules formant un tissu à des contraintes mécaniques ;

    **caractérisé en ce que** le champ magnétique appliqué varie dans une direction de translation linéaire de sorte qu'une force de translation linéaire soit appliquée par rapport aux cellules formant un tissu, et **en ce que** le matériau magnétique comprend des micro- ou nanoparticules magnétiques ou un ferrofluide.

2. Procédé selon la revendication 1, dans lequel le matériau magnétique est attaché aux cellules formant un tissu.

3. Procédé selon la revendication 1 ou la revendication 2, dans lequel le champ magnétique varie de manière sinusoïdale.

4. Procédé selon les revendications 1 à 3, dans lequel le champ magnétique varie à une fréquence de 0,1 à 10 Hz.

5. Procédé selon les revendications 1 à 4, dans lequel les cellules formant un tissu sont cultivées sur ou dans un échafaudage en 3 dimensions.

6. Procédé selon les revendications 1 à 5, dans lequel les cellules formant un tissu sont cultivées dans un bioréacteur (1) dans lequel circule un milieu de culture.

7. Procédé selon la revendication 6, dans lequel le champ magnétique est appliqué depuis l'extérieur du bioréacteur (1).

8. Procédé selon l'une quelconque des revendications 1 à 7, dans lequel les cellules qui sont cultivées sont destinées à former du tissu conjonctif.

9. Procédé selon la revendication 8, dans lequel les cellules qui sont cultivées sont destinées à former de l'os, du cartilage, un ligament ou des tendons.

10. Procédé selon l'une quelconque des revendications 1 à 9, dans lequel au moins deux types cellulaires différents sont cultivés.

11. Procédé selon la revendication 10, dans lequel les cellules qui sont cultivées comprennent des cellules formant de l'os et des cellules formant du cartilage.

12. Procédé selon l'une quelconque des revendications 1 à 11, dans lequel les forces appliquées aux cellules ou à l'échafaudage sont dans la plage de 0,1 à 100 pN.

13. Procédé selon la revendication 6, dans lequel le champ magnétique est généré à l'extérieur du récipient de culture tissulaire, où le champ magnétique est fourni par un aimant permanent (2) ou un électroaimant, où l'aimant permanent est déplacé par rapport aux cellules qui sont cultivées, un tel mouvement étant en direction longitudinale le long du bioréacteur (1) ou en s'approchant et en s'éloignant du bioréacteur (1).

14. Procédé selon la revendication 6, dans lequel les aimants permanents ou les électroaimants (2) sont positionnés à l'extérieur du bioréacteur (1) et sont espacés de manière longitudinale le long de celui-ci, les aimants (2) étant montés sur un arrangement de support qui est dirigé pour faire osciller les aimants (2) en direction transversale en s'approchant et en s'éloignant du bioréacteur (1).

15. Procédé selon la revendication 6, dans lequel les aimants permanents ou les électroaimants (2) sont positionnés à l'extérieur du bioréacteur (1) et sont espacés de manière longitudinale le long de celui-ci, les aimants (2) étant montés sur un arrangement de support qui est dirigé de sorte à faire osciller les aimants (2) en direction parallèle à l'axe longitudinal du bioréacteur (1).

**FIG. 1**

Nutrient Flow — 5

Tissue Constructs

Bioreactor Chamber

4

1

Permanent Magnets — 2

2

3

Sweep Frequency = 1 Hz

To Computer-controlled Drive System

6

Bone

Cartilage

Permanet Magnets

Sweep Frequency = 1 Hz

To Computer-controlled Drive System

Waste Out

Nutrient Flow

Bioreactor Chamber

Cartilage/Bone Polymer scaffolds

Cartilage
Bone

FIG.2

EP 1 343 872 B1

(a)

14

B = 0

11

13

10

Mechanosensitive
Ion Channel

Cell Membrane

Cytoplasm
(Cell·Interior)

12

(b)

14

B < 250mT

11

10

FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5486457 A

### Non-patent literature cited in the description

- **YUGE LOUIS et al.** *In Vitro Cell Dev. Biol. Animal,* June 2000, vol. 36 (6), 383-386
- **LIU et al.** *In Vitro Cell. Dev. Biol. - Anima,* February 1999, vol. 33, 87-93
- **KASPAR et al.** *Journal of Biomechanics,* 2000, vol. 33, 45-51
- **GLOGAUER et al.** *Pflügers Arch - Eur J Physiol,* 1998, vol. 435, 320-327
- **SMALT et al.** *Am. J. Physiol. Endocrinol. Metab.,* 1997, vol. 273, 751-758
- **WANG et al.** *Am. J. Physiol. Cell Physiol.,* 2000, vol. 279, 188-194
- **WANG.** *Hypertension,* 1998, vol. 32, 162-165
- **GLOGAUER et al.** *Am. J. Physiol.,* 1995, vol. 269, C1093-C1104
- **YING, Y. ; PEAK, M. ; MAGNAY, J. ; EL HAJ, A.J.** Dynamic cell scaffold interactions: implications for tissue engineering. *Proceedings of the second Smith and Nephew international symposium on tissue engineering,* 2000
- **EL HAJ, AJ ; LM WALKER ; MR PRESTON ; SJ PUBLICOVER.** Mechanotransduction pathways in bone: calcium fluxes and the role of voltage-operated calcium channels. *Med. Biol. Eng. Comp.,* 1999, vol. 37, 403-409
- **HOWARD J ; AJ HUDSPETH.** Compliance of the hair bundle associated with the gating of mechanoelectrical transduction channels in the bullfrog's saccular hair cell. *Neuron,* 1989, vol. 1, 189-199
- **WALKER et al.** *J Cell Biochem,* 2000
- **PARDOE, H ; W CHUA-ANUSORN ; TG ST. PIERRE ; J DOBSON.** Structural and magnetic properties of nanoscale magnetic particles synthesised by coprecipitation of iron oxide in the presence of dextran or polyvinyl alcohol. *J. Magn. Mag. Materials,* 2000
- **KIRSCHVINK, JL.** Comments on "Constraints on biological effects of weak extremely-low-frequency electromagnetic fields. *Phys. Rev.,* 1992, vol. 46, 2178-2184
- **DOBSON, J ; TG ST. PIERRE.** Application of the Ferromagnetic Transduction Model to D.C. and Pulsed Magnetic Fields: Effects on Epileptogenic Tissue and Implications for Cellular Phone Safety. *Biochem. Biophys. Res. Commun.,* 1996, vol. 227, 718-723